# EUROPEAN PATENT APPLICATION

(11) **EP 4 821 483 A1**
(43) Date of publication of application: **14.10.2026**
(21) Application number: 24900108.2
(22) Date of filing: 05.12.2024
(51) Int. Cl.: A61K 9/14, A61K 9/16, A61K 31/4436, A61K 9/20

(54) **SOLID DISPERSION INCLUDING HETEROCYCLE DERIVATIVE, METHOD FOR PREPARING SAME, AND PHARMACEUTICAL COMPOSITION INCLUDING SAME**

(30) Priority: 07.12.2023 KR 20230177109
(71) Applicant: JW Pharmaceutical Corporation, Gyeonggi-do 13840 (KR)
(72) Inventor: HWANG, Kyu-Mok, Seoul 05503 (KR); UM, Yun-Sik, Gwangmyeong-si, Gyeonggi-do 14306 (KR); CHIN, Seiho, Hwaseong-si, Gyeonggi-do 18307 (KR)
(74) Representative: HGF
(86) International application number: PCT/IB2024/062241
(87) International publication number: WO 2025/120552

(57) **Abstract**

The present invention relates to a heterocycle derivative-containing solid dispersion, a method for preparing the same, and a pharmaceutical composition including the same, in which the heterocycle derivative-containing solid dispersion may include a compound represented by formula 1, which is a STAT3 inhibitor, a stereoisomer thereof, or a pharmaceutically acceptable salt thereof as an active ingredient; and one polymer of hydroxypropyl cellulose (HPC), hydroxypropyl methylcellulose (HPMC), and a methacrylic acid-methyl methacrylate copolymer.

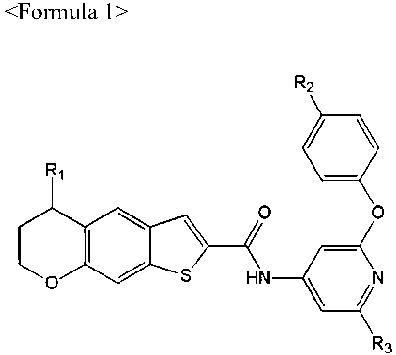

## Description

### [Technical Field]

The present invention relates to a heterocycle derivative-containing solid dispersion, a method for preparing the same, and a pharmaceutical composition including the same, and more particularly, to a heterocycle derivative-containing solid dispersion with improved bioavailability, a method for preparing the same, and a pharmaceutical composition including the same.

### [Background]

Signal transducer and activator of transcription (STAT) proteins are transcription factors which transmit signals from various extracellular cytokines and growth factors into nucleuses. So far, the STAT proteins have been known to have seven subtypes (that is, STAT1, STAT2, STAT3, STAT4, STAT5a, STAT5b, and STAT6) and generally consist of about 750 to 850 amino acids.

In particular, the STAT3 proteins are known to play a key role in the IL-6 and EGF signaling systems. It has also been reported that the STAT3 proteins are continuously activated in patients with solid cancer, which occurs in the prostate, stomach, breast, lung, pancreas, kidney, uterus, ovary, head and neck, etc., and patients with blood cancer such as acute and chronic leukemia, multiple myeloma, etc.

In many studies, STAT3 is considered a highly promising novel anticancer target, since apoptosis induced by anti-sensing STAT3 has been observed in a variety of cancer cell lines.

On the contrary, unlike STAT3, STAT1 has the same intracellular reaction pathway of cytokines and growth factors as that of STAT3, but it has been reported that STAT1 increases congenital and acquired immunity to suppress the proliferation of cancer cells or induce a pro-apoptotic response.

A heterocycle derivative compound as a STAT3 inhibitor, which has an excellent effect of selectively inhibiting STAT3 compared to STAT1 and exhibits an excellent growth inhibitory effect on various cancer cells, is disclosed in Korean laid-open publication No. 2017-0081708.

### [Related Art References]

### [Patent Documents]

Korean laid-open publication No. 2017-0081708

### [Disclosure]

### [Technical Problem]

One object of the present invention is to provide a heterocycle derivative-containing solid dispersion which is capable of improving the solubility of drugs and has improved physicochemical properties.

One object of the present invention is to provide a method for preparing the heterocycle derivative-containing solid dispersion.

One object of the present invention is to provide a pharmaceutical composition including the heterocycle derivative-containing solid dispersion.

### [Technical Solution]

A heterocycle derivative-containing solid dispersion for one object of the present invention may include a heterocycle derivative represented by a following formula 1, which is a STAT3 inhibitor, a stereoisomer thereof, or a pharmaceutically acceptable salt thereof as an active ingredient; and one polymer of hydroxypropyl cellulose (HPC), hydroxypropyl methylcellulose (HPMC), and a methacrylic acid-methyl methacrylate copolymer: 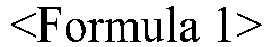 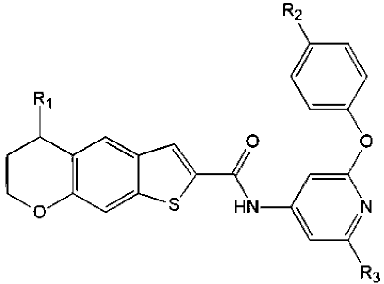
wherein in above formula 1,
R₁ is -S(=O)(=Ra)Rb,
Ra is =O or =NH,
Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
at least one H of Ra or Rb may be each independently substituted with F, Br, Cl or I, and
R₂ and R₃ are each independently F, Br, Cl or I.

In one embodiment, the heterocycle derivative represented by above formula 1 may be compound 1:

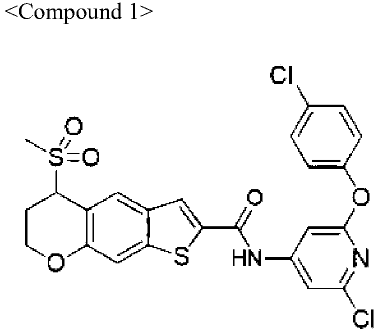

Compound 1 may be referred to as N-[2-chloro-6-(4-chlorophenoxy)pyridin-4-yl]-4-methylsulfonyl-3,4-dihydro-2H-thieno[3,2-g]chromen-7-carboxamide.

In one embodiment, the heterocycle derivative represented by above formula 1 may be any one of (4S)-N-[2-chloro-6-(4-chlorophenoxy)pyridin-4-yl]-4-methylsulfonyl-3,4-dihydro-2H-thieno[3,2-g]chromen-7-carboxamide, (4R)-N-[2-chloro-6-(4-chlorophenoxy)pyridin-4-yl]-4-methylsulfonyl-3,4-dihydro-2H-thieno[3,2-g]chromen-7-carboxamide, and a mixture thereof.

The pharmaceutically acceptable salt may mean a salt conventionally used in the pharmaceutical field, and is not particularly limited. For example, the pharmaceutically acceptable salt may be a salt with an inorganic acid or an organic acid, in which the inorganic acid may be hydrochloric acid, sulfuric acid, nitric acid, phosphoric acid, perchloric acid, bromic acid, and the like, and the organic acid may be acetic acid, methanesulfonic acid, ethanesulfonic acid, p-toluenesulfonic acid, fumaric acid, maleic acid, malonic acid, phthalic acid, succinic acid, lactic acid, citric acid, gluconic acid, tartaric acid, salicylic acid, malic acid, oxalic acid, benzoic acid, embonic acid, aspartic acid, glutamic acid, and the like. Organic bases which may be used in the preparation of organic base addition salts may include tris(hydroxymethyl)methylamine, dicyclohexylamine, and the like. Amino acids which may be used in the preparation of amino acid addition salts may include natural amino acids, for example, alanine and glycine.

In one embodiment, the heterocycle derivative represented by formula 1, the stereoisomer thereof, or the pharmaceutically acceptable salt thereof in the solid dispersion may be amorphous.

In one embodiment, the solid dispersion may be amorphous.

In one embodiment, as active ingredient, the heterocycle derivative represented by formula 1 according to the present invention, the stereoisomer thereof, or the pharmaceutically acceptable salt thereof and the polymer may have a weight ratio of 1:1 to 1:10. For example, the weight ratio may be 1:1 to 1:5 or 1:1 to 1:3.

In one embodiment, an average particle size of the heterocycle derivative-containing solid dispersion according to the present invention may be 5 um to 15 um. The average particle size in the present invention may be a volume-average diameter. The average particle size may be D50 and D50 may be defined as a particle size which occupies a cumulative 50% by volume assuming that the total particle amount is 100%. The average particle size may be measured by a laser diffraction method or a dynamic light scattering method. For example, the average particle size may be measured by a dry dispersion method. For example, the average particle size of the heterocycle derivative-containing solid dispersion according to the present invention may be 5 um to 10 um, 7 um to 9 um, 10 um to 15 um, or 13 um to 15 um.

In one embodiment, the solubility of the heterocycle derivative in the heterocycle derivative-containing solid dispersion according to the present invention may be at least 40 ug/mL or more, for example, at least 60 ug/mL or more in a pH 6.5 eluate. For example, the solubility of the heterocycle derivative in the heterocycle derivative-containing solid dispersion according to the present invention may be 60 ug/mL to 120 ug/mL in a FaSSIF pH 6.5 eluate.

In one embodiment of the present invention, the heterocycle derivative-containing solid dispersion according to the present invention may be for preventing or treating STAT3 protein activation-mediated diseases.

A method for preparing a heterocycle derivative-containing solid dispersion for one object of the present invention may include:
preparing a mixed solution including a heterocycle derivative represented by above formula 1, which is a STAT3 inhibitor, a stereoisomer thereof, or a pharmaceutically acceptable salt thereof; and one polymer of hydroxypropyl cellulose, hydroxypropyl methylcellulose, and a methacrylic acid-methyl methacrylate copolymer; and
preparing a solid dispersion from the mixed solution.

Accordingly, the heterocycle derivative-containing solid dispersion according to the present invention described above may be prepared.

The preparing the solid dispersion may be performed by a spray-drying method, a fluidized bed granulation method, a fluidized bed coating method, a rotary evaporation concentration method, a supercritical fluid method, or a melt extrusion method.

In one embodiment, the preparing the solid dispersion may be performed through the spray-drying method. For example, the preparing the solid dispersion may include: performing a spray-drying process on the mixed solution; and drying a solid obtained through the spray-drying process.

In one embodiment, the mixed solution may include a mixed solvent of dichloromethane and an alcohol having 1 to 5 carbon atoms, or dichloromethane. For example, the alcohol may be methanol or ethanol.

In one embodiment, the mixed solution may include a mixed solvent containing dichloromethane and an alcohol having 1 to 5 carbon atoms at a weight ratio of 6:4 to 9:1. For example, the weight ratio may be 7:3 to 9:1 or 7:3 to 8:2.

In one embodiment, the mixed solution may be prepared by mixing a solid content including a heterocycle derivative represented by above formula 1, a stereoisomer thereof, or a pharmaceutically acceptable salt thereof; and one polymer of hydroxypropyl cellulose, hydroxypropyl methylcellulose, and a methacrylic acid-methyl methacrylate copolymer, with a solvent. In this case, the solvent may include the mixed solvent or dichloromethane. The solid content and the solvent may have a weight ratio of 0.5:99.5 to 20:80 based on the total weight of 100 thereof. For example, the solid content and the solvent may have a weight ratio of 0.5:99.5 to 15:85, or 1:99 to 10:90.

A pharmaceutical composition for one object of the present invention may include a heterocycle derivative represented by above formula 1, which is a STAT3 inhibitor, a stereoisomer thereof, or a pharmaceutically acceptable salt thereof; one polymer of hydroxypropyl cellulose, hydroxypropyl methylcellulose, and a methacrylic acid-methyl methacrylate copolymer; and a pharmaceutically acceptable carrier.

In one embodiment, the pharmaceutical composition may be a mixture of a heterocycle derivative-containing solid dispersion including a heterocycle derivative represented by formula 1, a stereoisomer thereof, or a pharmaceutically acceptable salt thereof; and one polymer of hydroxypropyl cellulose, hydroxypropyl methylcellulose, and a methacrylic acid-methyl methacrylate copolymer, and the pharmaceutically acceptable carrier. In this case, the mixture may be a solid formulation, and may be, for example, a formulation of granule, tablet, capsule, dry syrup, or powder.

The pharmaceutically acceptable carrier included in the pharmaceutical composition of the present invention may include an excipient, a disintegrant, a glidant, a binder, and the like.

The excipient may include lactose, mannitol, sorbitol, microcrystalline cellulose, starch, pregelatinized starch, calcium phosphate, silicon dioxide (SiO₂), cellulose, sodium hydrogen carbonate, a mixture thereof, and the like. The disintegrant may include croscarmellose sodium, crospovidone, sodium starch glycolate, low-substituted hydroxypropyl cellulose, and the like. The glidant may include sodium stearyl fumarate, magnesium stearate, calcium stearate, talc, sodium lauryl sulfate, and the like. The binder may include glucose syrup, polyvinylpyrrolidone, polyethylene glycol 6000, methylcellulose, sodium carboxymethylcellulose, ethylcellulose, hydroxyethylcellulose, and the like. The pharmaceutically acceptable carrier may be appropriately selected and used by those skilled in the art according to the form of the pharmaceutical composition.

In one embodiment, the pharmaceutically acceptable carrier may include at least one selected from microcrystalline cellulose, colloidal silicon dioxide, croscarmellose sodium, crospovidone, sodium starch glycolate, polyvinylpyrrolidone vinyl acetate, sodium lauryl sulfate, magnesium stearate, and sodium stearyl fumarate.

The pharmaceutical composition according to the present invention may be an oral pharmaceutical composition.

The pharmaceutical composition may be in various forms, but may include a solid preparation of granule, tablet, capsule, dry syrup, powder, or the like, and may be, for example, a preparation of granule, tablet, or capsule. Such preparation may be prepared according to methods conventionally used in the pharmaceutical field.

In one embodiment, the tablet may be prepared by mixing the heterocycle derivative-containing solid dispersion with an excipient, a disintegrant, a glidant, or the like, or adding an excipient or the like to a heterocycle derivative-containing solid dispersion to prepare a granule, and then mixing with an excipient, a disintegrant, a glidant, or the like, followed by tableting. For example, the tablet may be a coated tablet coated with a film or an enteric coating on an uncoated tablet including the heterocycle derivative-containing solid dispersion, an excipient, a disintegrant, a glidant, etc. A coating agent may include polyvinyl alcohol, polyvinyl acetate, polyethylene glycol, titanium dioxide, iron oxide, Opadry^{®}, or the like.

In one embodiment, the capsule preparation may be prepared by filling a capsule with a mixture including an excipient, a disintegrant, a glidant, and the like together with a heterocycle derivative-containing solid dispersion. In this case, the mixture may be a granule.

The pharmaceutical composition according to the present invention may be a pharmaceutical composition for preventing or treating STAT3 protein activation-mediated diseases.

The present invention may also provide a method for preventing or treating STAT3 protein activation-mediated diseases, including administering an effective amount of the heterocycle derivative-containing solid dispersion according to the present invention or the pharmaceutical composition according to the present invention to a subject in need thereof.

The present invention may also provide a use of the heterocycle derivative-containing solid dispersion according to the present invention or the pharmaceutical composition according to the present invention for preventing or treating STAT3 protein activation-mediated diseases.

The present invention may also provide a use of the heterocycle derivative-containing solid dispersion according to the present invention or the pharmaceutical composition according to the present invention in preparing a medication for preventing or treating STAT3 protein activation-mediated diseases.

In one embodiment, in the present invention, the STAT3 protein activation-mediated diseases may include cancer. Specifically, the STAT3 protein activation-mediated diseases may include at least any one of solid cancer, blood cancer, radiation- or drug-resistant cancer, and metastatic cancer.

In one embodiment, in the present invention, the STAT3 protein activation-mediated diseases may include at least any one of breast cancer, lung cancer, stomach cancer, prostate cancer, uterine cancer, ovarian cancer, kidney cancer, pancreatic cancer, liver cancer, colon cancer, skin cancer, head and neck cancer, thyroid cancer, osteosarcoma, acute or chronic leukemia, multiple myeloma, B- or T-cell lymphoma, and non-Hodgkin's lymphoma.

In one embodiment of the present invention, a heterocycle derivative-containing solid dispersion of the present invention may include:
(1) a heterocycle derivative-containing solid dispersion including a heterocycle derivative represented by a following formula 1, a stereoisomer thereof, or a pharmaceutically acceptable salt thereof as an active ingredient; and
   one polymer of hydroxypropyl cellulose (HPC), hydroxypropyl methylcellulose (HPMC), and a methacrylic acid-methyl methacrylate copolymer: 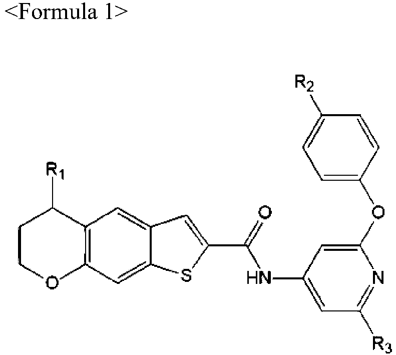
   wherein in above formula 1,
   R₁ is -S(=O)(=Ra)Rb,
   Ra is =O or =NH,
   Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
   at least one H of Ra or Rb may be each independently substituted with F, Br, Cl or I, and
   R₂ and R₃ are each independently F, Br, Cl or I.
(2) In (1), the heterocycle derivative represented by above formula 1 may be N-[2-chloro-6-(4-chlorophenoxy)pyridin-4-yl]-4-methylsulfonyl-3,4-dihydro-2H-thieno[3,2-g]chromen-7-carboxamide.
(3) In (1) or (2), the heterocycle derivative represented by formula 1 may be one of (4S)-N-[2-chloro-6-(4-chlorophenoxy)pyridin-4-yl]-4-methylsulfonyl-3,4-dihydro-2H-thieno[3,2-g]chromen-7-carboxamide, (4R)-N-[2-chloro-6-(4-chlorophenoxy)pyridin-4-yl]-4-methylsulfonyl-3,4-dihydro-2H-thieno[3,2-g]chromen-7-carboxamide, and a mixture thereof.
(4) In any one of (1) to (3), the heterocycle derivative represented by formula 1, the stereoisomer thereof, or the pharmaceutically acceptable salt thereof may be amorphous.
(5) In any one of (1) to (4), the heterocycle derivative represented by formula 1, the stereoisomer thereof, or the pharmaceutically acceptable salt thereof and the polymer may have a weight ratio of 1:1 to 1:10.
(6) In any one of (1) to (5), an average particle size may be 5 um to 15 um.

In one embodiment of the present invention, a method for preparing a heterocycle derivative-containing solid dispersion of the present invention may include:
(7) preparing a mixed solution including a heterocycle derivative represented by a following formula 1, a stereoisomer thereof, or a pharmaceutically acceptable salt thereof; and one polymer of hydroxypropyl cellulose, hydroxypropyl methylcellulose, and a methacrylic acid-methyl methacrylate copolymer; and
   preparing a solid dispersion from the mixed solution: 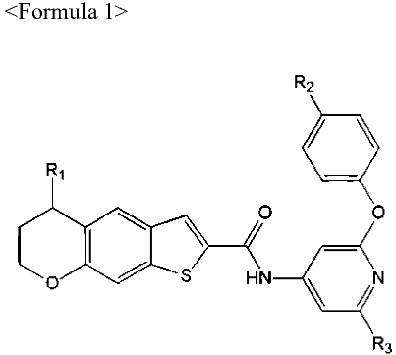
   wherein in above formula 1,
   R₁ is -S(=O)(=Ra)Rb,
   Ra is =O or =NH,
   Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
   at least one H of Ra or Rb may be each independently substituted with F, Br, Cl or I, and
   R₂ and R₃ are each independently F, Br, Cl or I.
(8) In (7), the preparing the solid dispersion may be performed through a spray-drying method.
(9) In (7) or (8), the mixed solution may include a mixed solvent of dichloromethane and an alcohol having 1 to 5 carbon atoms, or dichloromethane.
(10) In any one of (7) to (9), the mixed solution may include a mixed solvent including dichloromethane and alcohol at a weight ratio of 6:4 to 9:1.

In one embodiment of the present invention, a pharmaceutical composition of the present invention may be an oral pharmaceutical composition including:
(11) a heterocycle derivative represented by a following formula 1, a stereoisomer thereof, or a pharmaceutically acceptable salt thereof as an active ingredient;
   one polymer of hydroxypropyl cellulose (HPC), hydroxypropyl methylcellulose (HPMC), and a methacrylic acid-methyl methacrylate copolymer: and
   a pharmaceutically acceptable carrier: 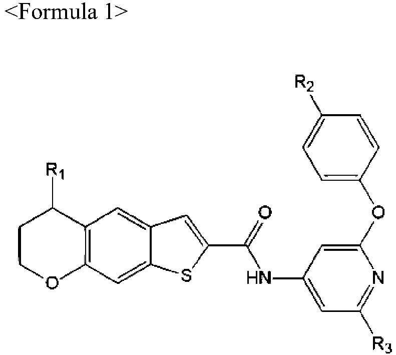
   wherein in above formula 1,
   R₁ is -S(=O)(=Ra)Rb,
   Ra is =O or =NH,
   Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
   at least one H of Ra or Rb may be each independently substituted with F, Br, Cl or I, and
   R₂ and R₃ are each independently F, Br, Cl or I.
(12) In (11), the pharmaceutically acceptable carrier may include at least one selected from an excipient, a disintegrant, a glidant, and a binder.
(13) In (11) or (12), the oral pharmaceutical composition may be a mixture of a heterocycle derivative-containing solid dispersion including the compound represented by formula 1, a stereoisomer thereof, or a pharmaceutically acceptable salt thereof; and one polymer of hydroxypropyl cellulose, hydroxypropyl methylcellulose, and a methacrylic acid-methyl methacrylate copolymer, and the pharmaceutically acceptable carrier.
(14) In any one of (11) to (13), the pharmaceutically acceptable carrier may include at least one selected from microcrystalline cellulose, colloidal silicon dioxide, croscarmellose sodium, crospovidone, sodium starch glycolate, polyvinylpyrrolidone vinyl acetate, sodium lauryl sulfate, magnesium stearate, and sodium stearyl fumarate.
(15) In any one of (11) to (14), the pharmaceutically acceptable carrier may include microcrystalline cellulose, colloidal silicon dioxide, croscarmellose sodium, and sodium stearyl fumarate.
(16) In any one of (11) to (15), the oral pharmaceutical composition may be any one solid preparation of granules, tablets, capsules, dry syrups, and powders.
(17) In any one of above (11) to (16), the oral pharmaceutical composition may be for preventing or treating STAT3 protein activation-mediated diseases.

In one embodiment of the present invention, (18) method for preventing or treating of the present invention may be a method for preventing or treating STAT3 protein activation-mediated diseases, including administering an effective amount of a heterocycle derivative-containing solid dispersion according to any one of above (1) to (6) or an oral pharmaceutical composition according to any one of above (11) to (16) to a subject in need thereof.

In one embodiment of the present invention, (19) a use of the present invention may be a use of a heterocycle derivative-containing solid dispersion according to any one of above (1) to (6) or an oral pharmaceutical composition according to any one of above (11) to (16) for preventing or treating STAT3 protein activation-mediated diseases.

In one embodiment of the present invention, (20) a use of the present invention may be a use of a heterocycle derivative-containing solid dispersion according to any one of above (1) to (6) or an oral pharmaceutical composition according to any one of above (11) to (16) for preparing a medication in preventing or treating STAT3 protein activation-mediated diseases.

### [Advantageous Effects]

According to a heterocycle derivative-containing solid dispersion of the present invention, a method for preparing the same, and a pharmaceutical composition including the same, a heterocycle derivative-containing solid dispersion with improved solubility of a STAT3 inhibitor may be provided. In addition, the pharmaceutical composition including the heterocycle derivative-containing solid dispersion may have excellent stability and dissolution properties. Accordingly, the bioavailability of the STAT3 inhibitor may be improved.

### [Description of Drawings]

FIGS. 1 and 2 are views showing PXRD analysis graphs of solid dispersions according to embodiments of the present invention.
FIGS. 3 and 4 are views showing the results of analyzing the solubility of solid dispersions according to embodiments of the present invention.
FIG. 5 is a view showing a graph of dissolution rates of each of an uncoated tablet and a coated tablet according to the present invention.

### [Mode for Invention]

Terms used in the present application are used only to describe a certain example and are not intended to limit the present invention. The singular expressions include the plural expressions unless the context clearly indicates otherwise. In the present application, terms such as "include," "have" or the like shall be intended to designate a presence of features, steps, operations, components, parts or combinations thereof described herein, and shall not be construed to exclude a possible presence or addition of one or more other features, steps, operations, components, parts or combinations thereof in advance.

All the terms used herein including technical or scientific terms have the same meaning as commonly understood by those ordinary skilled in the art, to which the present invention pertains, unless defined otherwise. Such terms as those defined in a generally used dictionary are to be interpreted to have the meanings equal to the contextual meanings in the relevant art, and are not to be interpreted to have ideal or excessively formal meanings, unless clearly defined in the present application.

Hereinafter, the present invention will be described in more detail with reference to embodiments. Since the present invention may have various modifications and various forms, specific embodiments is not intended to limit the specific disclosed forms.

### Synthesis example 1 - Synthesis of compound 1a

(4S)-N-[2-chloro-6-(4-chlorophenoxy)pyridin-4-yl]-4-methylsulfonyl-3,4-dihydro-2H-thieno[3,2-g]chromen-7-carboxamide as compound 1a was synthesized by substantially the same process as a method described in Example 43 of Korean laid-open publication No. 2017-0081708.

¹H-NMR(400MHz, DMSO-d₆): *δ* 10.96 (s, 1H), 8.31 (s, 1H), 8.06 (s, 1H), 7.66 (s, 1H), [0633] 7.58 (s, 1H), 7.52 (d, 2H, J=8.80Hz), 7.31 (s, 1H), 7.27 (d, 2H, J=8.80Hz), 4.82 (m, 1H), 4.45-4.51 (m, 1H), 4.30-4.34 (m, 1H), 3.16 (s, 3H), 2.60-2.64 (m, 1H), 2.29-2.39 (m, 1H); LC/MS ESI (+): 549 (M+1).

### Examples 1 to 8 - Preparation of solid dispersion

A mixed solvent was prepared by setting a volume ratio of dichloromethane and methanol to 8:2, compound 1a and a polymer were dissolved in the mixed solvent prepared according to the components and compositions shown in table 1 below, and then a solid dispersion was prepared using a spray dryer (Buchi B-290 Spray Dryer & B-295 Inert Loop Unit). The secondary drying was performed for about 20 hours in a vacuum oven at a temperature of 40°C under a negative pressure of 300 mbar.

**[Table 1]**

| Component | **Example 1** | **Example 2** | **Example 3** | **Example 4** | **Example 5** | **Example 6** | **Example 7** | **Example 8** |
|---|---|---|---|---|---|---|---|---|
| Polymer | HPC (SL) | HPC (SL) | HPC (SL) | HPMC (E3) | HPMC (E3) | HPC (SL) | Eudragit (L100) | Eudragit (L100) |
| Solid content | 8 wt% | 8 wt% | 8 wt% | 8 wt% | 8 wt% | 6 wt% | 6 wt% | 4 wt% |
| Weight ratio of compound 1a and polymer | 1:1 | 1:2 | 1:3 | 1:1 | 1:2 | 1:2 | 1:1 | 1:1 |

### Evaluation 1 - Crystal form

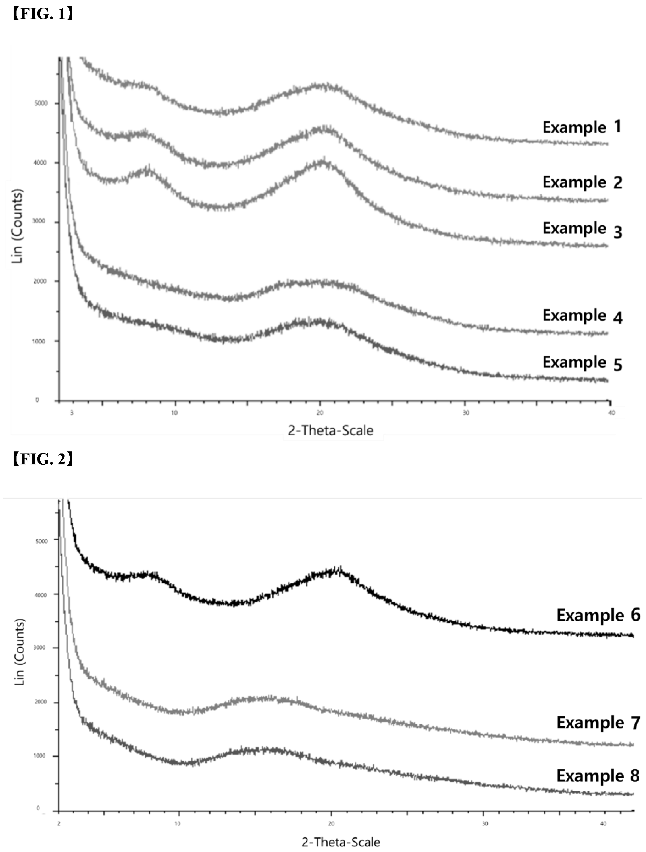

PXRD analysis was performed on each of the solid dispersions according to Examples 1 to 8, and the results thereof are shown in FIGS. 1 and 2.

FIGS. 1 and 2 are views showing PXRD analysis graphs of solid dispersions according to embodiments of the present invention. Referring to FIGS. 1 and 2, it can be confirmed that both the solid dispersion according to the present invention and compound 1a are amorphous.

### Evaluation 2 - DSC

DSC evaluation was performed on each of the solid dispersions according to Examples 1 to 8, and Tg values obtained therefrom are shown in table 2 below.

**[Table 2]**

| **Classification** | **Glass transition temperature (°C)** |
|---|---|
| **Example 1** | T_{g}=129 |
| **Example 2** | T_{g}=71 |
| **Example 3** | T_{g}=76 |
| **Example 4** | T_{g}=126 |
| **Example 5** | T_{g}=127 (108) |
| **Example 6** | T_{g}=84 |
| **Example 7** | T_{g}=131 |
| **Example 8** | T_{g}=132 |

Referring to table 2, it can be confirmed that the solid dispersion according to the present invention exhibits a single glass transition temperature, even if except for Example 5 in which a main glass transition mainly occurs at 127°C and a weak thermal change occurs at 108°C. It means that the solid dispersion according to the present invention has high miscibility, and indicates that stability is excellent.

### Evaluation 3 - Solubility

For the obtained solid dispersion, the solubility was confirmed in a FaSSIF pH 6.5 eluate using a Pion Micro Diss instrument. Measuring instrument and settings are shown in table 3 below.

**[Table 3]**

| **Instrument type** | Pion Micro-dissolution system |
|---|---|
| **Stir Speed** | 250 rpm |
| **Media volume** | 20 mL |
| **Temperature** | 37±2 °C |
| **Dissolution time (dissolution testing)** | 24 hr |
| **Sampling rate (dissolution testing)** | 1. Measured every 30 seconds until 30 minutes |
| | 2. Measured every 5 minutes thereafter and until 5 hours |
| | 3. Measured every 30 minutes thereafter and until 24 hours |

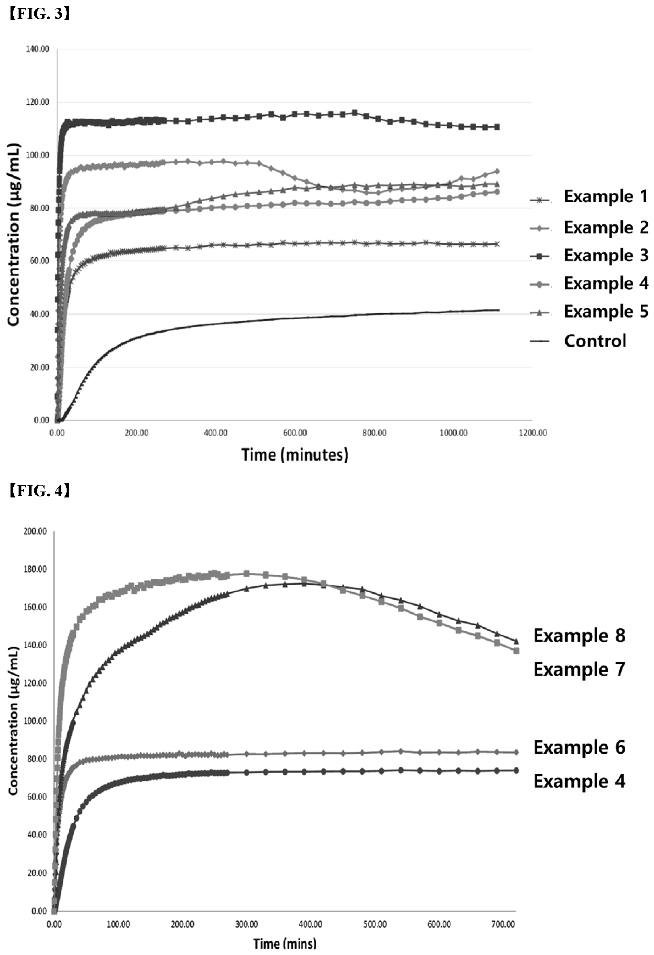

After 12 hours, the concentration (µg/mL) of compound 1a dissolved in the FaSSIF pH 6.5 eluate is shown in Table 4 below, FIGS. 3 and 4.

**[Table 4]**

| **Example 1** | **Example 2** | **Example 3** | **Example 4** | **Example 5** | **Control group (API only)** |
|---|---|---|---|---|---|
| 66.73 | 86.85 | 115.35 | 81.68 | 88.16 | 39.16 |

Referring to Table 4 and FIGS. 3 and 4, it can be confirmed that the solubility of compound 1a is improved by at least 1.7 times or more and up to 4 times or more by the solid dispersion according to the present invention.

### Example 9 - Preparation of composition

A solid dispersion was prepared using the same polymer type, the same weight ratio of compound 1a and the polymer, the same mixed solvent, and the same solid content as described in Example 4, and the solid dispersion was used to prepare samples 1 to 3 as compositions containing the solid dispersion according to the components and contents of Table 5 below. In Table 5, the unit of each content is w/w%, which means a weight of each component based on total 100% by weight of the tablet.

**[Table 5]**

| **Component name** | **Mixing purpose** | **Content (w/w%)** | | |
|---|---|---|---|---|
| | | **Sample 1** | **Sample 2** | **Sample 3** |
| **Solid dispersion** | Main component | 30 | 30 | 30 |
| **Microcrystalline cellulose** | Excipient | 63 | 63 | 58 |
| **Colloidal sili con dioxide** | Fluidizing agent | - | 1 | - |
| **Croscarmellose sodium** | Disintegrant | - | | 5 |
| **Crospovidone** | Disintegrant | - | 5 | - |
| **Sodium starch glycolate** | Disintegrant | 5 | - | - |
| **Polyvinylpyrrolidone vinyl acetate (PVPVA 64F)** | Binder | - | - | 6 |
| **Sodium lauryl sulfate** | Surfactant | 1 | - | - |
| **Magnesium stearate** | Glidant | - | 1 | - |
| **Sodium stearyl fumarate** | Glidant | 1 | - | 1 |
| **Total** | | 100 | 100 | 100 |

### Evaluation 4 - Stability (recrystallization/related substance)

For each of samples 1 to 3, related substances were measured through HPLC analysis compared to an initial after two and four weeks of accelerated (40°C/75% RH) and temperature stress conditions (60°C), and it was confirmed through XRPD analysis whether an active ingredient was recrystallized and an amorphous form was lost. The results of measuring related substances are shown in Table 6 below.

**[Table 6]**

| **Evaluation Time point** | **Storage condition** | **Total related substance (% a/a)** | | |
|---|---|---|---|---|
| | | **Sample 1** | **Sample 2** | **Sample 3** |
| Initial | | 0.4 | 0.4 | 0.4 |
| 2 weeks | 40°C/75% RH open | 0.4 | 0.4 | 0.4 |
| 2 weeks | 60°C open | 0.5 | 0.4 | 0.4 |
| 4 weeks | 40°C/75% RH open | 0.5 | 0.6 | 0.6 |
| 4 weeks | 60°C open | 0.6 | 0.5 | 0.6 |

Referring to Table 6, it can be confirmed that a related substance is not substantially generated even under each of accelerated and stress conditions. In addition, even in the XRPD analysis results, it was confirmed that recrystallization of the active ingredient does not occur and an amorphous form is maintained as it is even after two and four weeks.

### Example 10 - Preparation of tablet

A solid dispersion was prepared using the same polymer type, the same weight ratio of compound 1a and the polymer, the same mixed solvent, and the same solid content as described in Example 4, and the solid dispersion was used to prepare a tablet containing the components and contents of Table 7 below. In Table 7 below, the unit of each content is w/w%, which means a weight of each component based on total 100% by weight of the tablet, and 50 w/w% of the solid dispersion means that the content of compound 1a in 800 mg of the total weight of the tablet is 200 mg.

**[Table 7]**

| **Classification** | **Component name** | **Mixing purpose** | **Content (w/w%)** |
|---|---|---|---|
| **Intragranular** | Solid dispersion | Main component | 50.0 |
| | Microcrystalline cellulose | Excipient | 42.5 |
| | Croscarmellose sodium | Disintegrant | 4.0 |
| | Sodium stearyl fumarate | Glidant | 0.5 |
| **Post-mixing** | Croscarmellose sodium | Disintegrant | 2.0 |
| | Sodium stearyl fumarate | Glidant | 0.5 |
| | Colloidal silicon dioxide | Glidant | 0.5 |
| Total | | | 100 |

The order of preparing the tablet was as follows.
(1) A non-lubricated mixture was sieved in an 850 µm sieve and added into a mixing vessel as follows. Materials were layered in the mixing vessel (drum blender) as follows and mixed at 30 rpm for 15 minutes. The order of adding each of components into the mixing vessel as a non-lubricated mixture is as follows: An excipient (microcrystalline cellulose) was weighed in an amount of 33% and added, the solid dispersion was weighed in an amount of 50% and added, an intragranular disintegrant (croscarmellose sodium) was weighed in an amount of 50% and added, and an excipient (microcrystalline cellulose) was weighed in an amount of 33% and added. Then, the solid dispersion was weighed in an amount of 50% and added, the intragranular disintegrant was weighed in an amount of 50% and added, and then the excipient (microcrystalline cellulose) was weighed in an amount of 33% and added. (the "intragranular component" x% means x% of the components included in the granules, assuming that the components included in the granules shown in Table 7 are 100%).
(2) An intragranular glidant (sodium stearyl fumarate) was sieved through a 500 µm sieve and added into the mixing vessel, and then mixed at 30 rpm for three minutes, after which the granules were prepared by a dry granulation process.
(3) A post-mixing disintegrant (croscarmellose sodium) was added and mixed at 30 rpm for six minutes, and then a post-mixing glidant (sodium stearyl fumarate) was added and mixed at 30 rpm for three minutes.
(4) An uncoated tablet was prepared by tableting at 10 rpm with a target mass of 800±5% and a hardness of 20±5 kP using a rotary tablet press.
(5) A coated tablet was prepared by coating the uncoated tablet prepared as above with Opadry QX yellow (321A220055).

### Evaluation 5 - Dissolution rate test

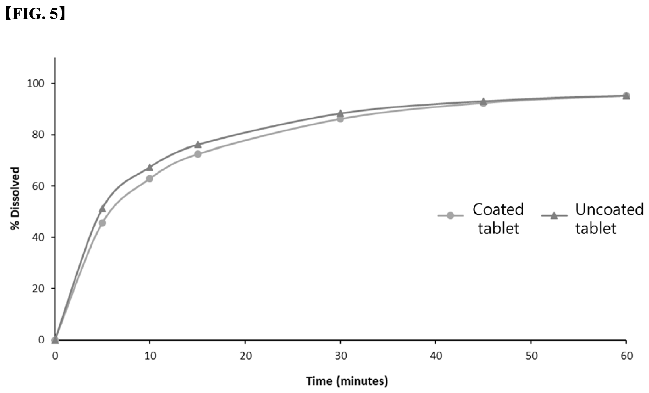

The dissolution rate of each of the uncoated tablet and coated tablet prepared as above was evaluated, and the evaluation method was performed according to Table 8 below. The results thereof were shown in FIG. 5.

**[Table 8]**

| **Dissolution specification** | **Detailed condition** |
|---|---|
| **Dissolution test method** | USP II (paddled method) |
| **Paddle speed** | 75 rpm ± 4 rpm |
| **Dissolution test solution** | pH 6.8 phosphate buffer + 0.3% w/v CTAB |
| **Eluate volume** | 900 mL |
| **Eluate temperature** | 37°C ± 0.5°C |
| **Sinker use or non-use** | Unused |
| **Sample collection amount** | 5 mL |
| **Sampling filter** | 0.45 µm Nylon membrane |
| **Cannula filter** | 10 µm |
| **Dissolution rate measurement time** | 5, 10, 15, 30, 45, 60, 75 (200 rpm) |
| **Test time** | 60 min + 15 min infinity spin |
| **Analysis method** | HPLC-UV |

Referring to FIG. 5, it can be confirmed that both the uncoated tablet and coated tablet exhibit an excellent dissolution rate. In particular, both the uncoated tablet and coated tablet prepared as described above exhibit substantially the same pattern as the dissolution rate of the solid dispersion per se.

The present invention has been described with reference to preferred exemplary embodiments herein, but it will be understood by those skilled in the art that the present invention may be variously changed and modified without departing from the spirit and field of the present invention, as described in the following scope of patent claims.

## Claims

1. A heterocycle derivative-containing solid dispersion comprising a heterocycle derivative represented by a following formula 1, a stereoisomer thereof, or a pharmaceutically acceptable salt thereof as an active ingredient; and
one polymer of hydroxypropyl cellulose (HPC), hydroxypropyl methylcellulose (HPMC), and a methacrylic acid-methyl methacrylate copolymer: 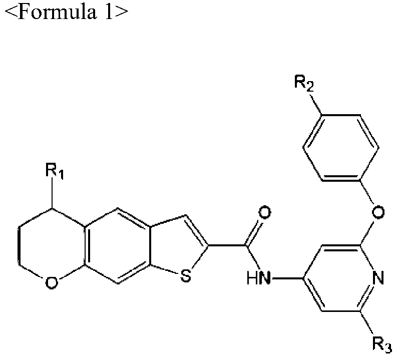
wherein in above formula 1,
R₁ is -S(=O)(=Ra)Rb,
Ra is =O or =NH,
Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
at least one H of Ra or Rb may be each independently substituted with F, Br, Cl or I, and
R₂ and R₃ are each independently F, Br, Cl or I.

2. The heterocycle derivative-containing solid dispersion according to claim 1, wherein the heterocycle derivative represented by formula 1 is N-[2-chloro-6-(4-chlorophenoxy)pyridin-4-yl]-4-methylsulfonyl-3,4-dihydro-2H-thieno[3,2-g]chromen-7-carboxamide.

3. The heterocycle derivative-containing solid dispersion according to claim 1, wherein the heterocycle derivative represented by formula 1 is one of (4S)-N-[2-chloro-6-(4-chlorophenoxy)pyridin-4-yl]-4-methylsulfonyl-3,4-dihydro-2H-thieno[3,2-g]chromen-7-carboxamide, (4R)-N-[2-chloro-6-(4-chlorophenoxy)pyridin-4-yl]-4-methylsulfonyl-3,4-dihydro-2H-thieno[3,2-g]chromen-7-carboxamide, and a mixture thereof.

4. The heterocycle derivative-containing solid dispersion according to claim 1, wherein the heterocycle derivative represented by formula 1, the stereoisomer thereof, or the pharmaceutically acceptable salt thereof is amorphous.

5. The heterocycle derivative-containing solid dispersion according to claim 1, wherein the heterocycle derivative represented by formula 1, the stereoisomer thereof, or the pharmaceutically acceptable salt thereof and the polymer have a weight ratio of 1:1 to 1:10.

6. The heterocycle derivative-containing solid dispersion according to claim 1, wherein an average particle size is 5 um to 15 um.

7. A method for preparing a heterocycle derivative-containing solid dispersion, the method comprising:
preparing a mixed solution including a heterocycle derivative represented by a following formula 1, a stereoisomer thereof, or a pharmaceutically acceptable salt thereof; and one polymer of hydroxypropyl cellulose, hydroxypropyl methylcellulose, and a methacrylic acid-methyl methacrylate copolymer; and
preparing a solid dispersion from the mixed solution: 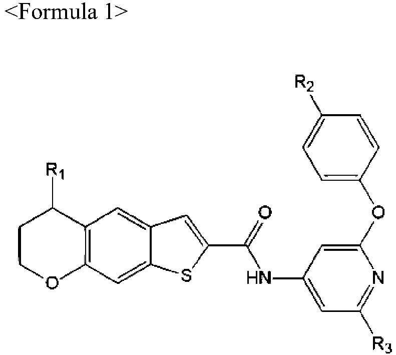
wherein in above formula 1,
R₁ is -S(=O)(=Ra)Rb,
Ra is =O or =NH,
Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
at least one H of Ra or Rb may be each independently substituted with F, Br, Cl or I, and
R₂ and R₃ are each independently F, Br, Cl or I.

8. The method according to claim 7, wherein the preparing the solid dispersion is performed through a spray-drying method.

9. The method according to claim 7, wherein the mixed solution comprises a mixed solvent of dichloromethane and an alcohol having 1 to 5 carbon atoms, or dichloromethane.

10. The method according to claim 7, wherein the mixed solution comprises a mixed solvent including dichloromethane and an alcohol having 1 to 5 carbon atoms at a weight ratio of 6:4 to 9:1.

11. An oral pharmaceutical composition comprising a heterocycle derivative represented by a following formula 1, a stereoisomer thereof, or a pharmaceutically acceptable salt thereof as an active ingredient;
one polymer of hydroxypropyl cellulose (HPC), hydroxypropyl methylcellulose (HPMC), and a methacrylic acid-methyl methacrylate copolymer: and
a pharmaceutically acceptable carrier: 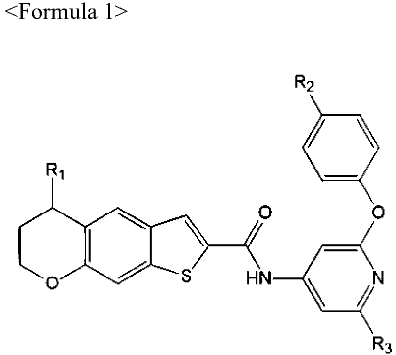
wherein in above formula 1,
R₁ is -S(=O)(=Ra)Rb,
Ra is =O or =NH,
Rb is H, C1-6 alkyl, C1-6 alkoxy-C1-6 alkyl, C1-6 alkylcarbonyl-C1-6 alkyl, C2-7 alkenyl, amino, or amino C1-6 alkyl,
at least one H of Ra or Rb may be each independently substituted with F, Br, Cl or I, and
R₂ and R₃ are each independently F, Br, Cl or I.

12. The oral pharmaceutical composition according to claim 11, wherein the pharmaceutically acceptable carrier comprises at least one selected from an excipient, a disintegrant, a glidant, and a binder.

13. The oral pharmaceutical composition according to claim 11, wherein the oral pharmaceutical composition is a mixture of a heterocycle derivative-containing solid dispersion including the compound represented by formula 1, the stereoisomer thereof, or the pharmaceutically acceptable salt thereof; and one polymer of hydroxypropyl cellulose, hydroxypropyl methylcellulose, and methacrylic acid-methyl methacrylate copolymer, and the pharmaceutically acceptable carrier.

14. The oral pharmaceutical composition according to claim 11, wherein the pharmaceutically acceptable carrier comprises at least one selected from microcrystalline cellulose, colloidal silicon dioxide, croscarmellose sodium, crospovidone, sodium starch glycolate, polyvinylpyrrolidone vinyl acetate, sodium lauryl sulfate, magnesium stearate, and sodium stearyl fumarate.

15. The oral pharmaceutical composition according to claim 11, wherein the pharmaceutically acceptable carrier comprises microcrystalline cellulose, colloidal silicon dioxide, croscarmellose sodium, and sodium stearyl fumarate.

16. The oral pharmaceutical composition according to claim 11, wherein the oral pharmaceutical composition is any one solid preparation of granules, tablets, capsules, dry syrups, and powders.

17. The oral pharmaceutical composition according to any one of claims 11 to 16, wherein the oral pharmaceutical composition is for preventing or treating STAT3 protein activation-mediated diseases.

18. A method for preventing or treating STAT3 protein activation-mediated diseases, the method comprising administering an effective amount of a heterocycle derivative-containing solid dispersion according to any one of claims 1 to 6 or an oral pharmaceutical composition according to any one of claims 11 to 16 to a subject in need thereof.

19. A use of a heterocycle derivative-containing solid dispersion according to any one of claims 1 to 6 or an oral pharmaceutical composition according to any one of claims 11 to 16 for preventing or treating STAT3 protein activation-mediated diseases.

20. A use of a heterocycle derivative-containing solid dispersion according to any one of claims 1 to 6 or an oral pharmaceutical composition according to any one of claims 11 to 16 for preparing a medication in preventing or treating STAT3 protein activation-mediated diseases.
